# EUROPÄISCHE PATENTSCHRIFT

(11) **EP 3 391 920 B1**
(45) Veröffentlichungstag und Bekanntmachung des Hinweises auf die Patenterteilung: **18.11.2020**
(21) Anmeldenummer: 18000362.6
(22) Anmeldetag: 16.04.2018
(51) Int. Cl.: A61M 5/158, A61M 5/32, A61M 25/06, A61M 39/02, A61M 25/00, A61M 5/142, A61M 25/02, A61M 39/10

(54) **APPLIZIERVORRICHTUNG ZUM PERKUTANEN EINFÜHREN EINER KANÜLE IN EINEN IMPLANTIERTEN PORT**
APPLICATOR FOR PERCUTANEOUS INSERTION OF A CANNULA INTO AN IMPLANTED PORT
DISPOSITIF D'APPLICATION DESTINÉ À L'INTRODUCTION PERCUTANÉE D'UNE CANULE DANS UN PORT IMPLANTÉ

(30) Priorität: 20.04.2017 DE 102017003808
(43) Veröffentlichungstag der Anmeldung: 24.10.2018
(73) Patentinhaber: Acti-Med AG, 36399 Freiensteinau (DE)
(72) Erfinder: Schwalb, Andreas, 63636 Brachttal (DE); Weisbecker, Ralf, 63619 Bad Orb (DE)
(74) Vertreter: Hase, Stephan

(56) Entgegenhaltungen:
- EP-B1- 2 173 411
- DE-A1- 10 333 118
- DE-A1-102007 011 303
- US-B1- 6 238 375

## Beschreibung

Bei derartigen Appliziervorrichtungen wird die Kanüle so weit in den implantierten Port eingeführt, dass der Boden des Körpers der Appliziervorrichtung auf der Haut des Patienten aufliegt. Wenn der Injektionsvorgang beendet ist, besteht die Notwendigkeit, die Kanüle so sicher aus dem Körper des Patienten zu entfernen, dass Verletzungen oder Verunreinigungen vermieden werden. Aus diesem Grund ist es bekannt, dass die Kanüle nach dem Gebrauch aus dem Körper des Patienten direkt in den Körper der Appliziervorrichtung zurückgezogen wird. Die DE 10 2007 011 303 A1, die DE 103 33 118 A1 und die US 6,238,375 B1 offenbaren Verfahren, bei denen die Extraktion der Kanüle aus dem Körper des Patienten in die Appliziervorrichtung manuell erfolgt. Dabei sieht die DE 10 2007 011 303 A1 ein Griffteil vor, welches zwei Flügel gegen eine Drehbeweglichkeit sichert und abnehmbar ist. Nach Entfernung des Griffteils können die beiden Flügel in eine senkrechte Position verdreht werden, wodurch dann die Kanüle aus dem Körper des Patienten in die Appliziereinrichtung gezogen werden kann.

In der EP 2 173 411 B1 wird eine Vorrichtung beschrieben, die aus einem Basiselement 10 und einem Zugelement 12 besteht, welches gegen das Basiselement vorgespannt ist. Die Vorspanneinrichtung besteht aus einer Druckfeder 40, an einem plattenförmigen Element (Betätigungselement) 30, angeordneten Riegelelementen 36 sowie Gegenstücken 38, die an der Unterseite des Zugelements 12 gebildet sind. Im vorgespannten Zustand sind die Riegelelemente und die Gegenstücke gegeneinander verhakt. In diesem Zustand ragt die am Zugelement befestigte Kanüle 14 durch die Öffnung 15 des Basiselements hindurch.

Das Betätigungselement ist am Boden des Basiselements in Querrichtung zur Kanülenlängsachse verschieblich angeordnet. Wird das Betätigungselement in Richtung auf die Kanüle verschoben, so wird die Verriegelung von Riegelelementen und Gegenstücken gelöst, die Druckfeder kann das Zugelement hochdrücken und damit die Kanüle in den Raum zwischen Zugelement und Basiselement zurückziehen. Wird das Betätigungselement weiter in Richtung auf die Kanüle verschoben, so verschließt das Betätigungselement die Öffnung im Boden des Basiselements und verhindert ein abermaliges Austreten der Kanülenspitze. Nachteilig an dieser Vorrichtung ist es, dass das Betätigungselement nicht gegen ein unbeabsichtigtes Verschieben gesichert ist. Im Gebrauch der Appliziervorrichtung kann jederzeit unbeabsichtigt eine Verschiebung des Betätigungselements erfolgen, wodurch der bestimmungsgemäße Gebrauch beeinträchtigt wird. Außerdem wird in der EP 2 173 411 B1 als Vorteil der Erfindung beschrieben, dass die Bedienung der Appliziervorrichtung mit nur einer Hand erfolgen könne. Durch eine solche Einhandbedienung wird nicht nur die Gefahr eines unbeabsichtigten Drucks auf das Betätigungselement erhöht, da es von der Kontrolle und Fingerfertigkeit des Anwenders abhängt, ob eine bestimmungsgemäße Anwendung erfolgt. Durch die Bedienung mit nur einer Hand wird beim Verschieben des Betätigungselements durch den drückenden Finger ein Druck quer zur Längsachse der Kanüle ausgeübt, der von den anderen die Appliziervorrichtung haltenden Fingern ausgeglichen werden muss. Wenn dies nicht oder nicht genügend gelingt, ist eine Instabilität der Kanülenlage die Folge.

Es ist Aufgabe der vorliegenden Erfindung, eine Appliziervorrichtung zum perkutanen Einführen einer Kanüle in einen implantierten Port bereitzustellen, die gegen ein unbeabsichtigtes Lösen der Vorspanneinrichtung gesichert und problemlos mit einer Hand zu bedienen ist.

Diese Aufgabe wird mit den Merkmalen des Anspruchs 1 gelöst. Vorteilhafte Ausbildungen ergeben sich aus den Unteransprüchen 2, 3 und 4.

Erfindungsgemäß sieht die Appliziervorrichtung ein an sich bekanntes Griffteil vor, mit dem die Vorrichtung nicht nur angewendet werden kann, sondern welches auch verhindert, dass die Vorspanneinrichtung unbeabsichtigt gelöst wird. Der zum Lösen der Vorspanneinrichtung vorgesehene Flügel kann erst aktiviert werden, nachdem das Griffteil entfernt worden ist. Der Flügel wird beim Lösen der Vorspanneinrichtung um eine Drehachse verschwenkt. Das untere Ende des Flügels drückt in Richtung der Längsachse der Kanüle die Rastnasen der Verrasterungseinrichtung auseinander und verhindert damit eine ungewünschte Krafteinwirkung quer zur Längsachse der Kanüle. So bleibt die Stabilität der Position der Kanüle gewahrt.

Im Aufnahmeteil der erfindungsgemäßen Vorrichtung ist eine Verschlussvorrichtung vorgesehen, die gegen die Kanüle vorgespannt ist. Sobald die Kanülenspitze in den Körper der Vorrichtung hineingezogen worden ist und die Bodenöffnung des Aufnahmeteils freigibt, wird diese Öffnung durch die vorgespannte Verschlussvorrichtung selbsttätig verschlossen. Eines manuellen Einwirkens vermittels eines Verschlussteils bedarf es nicht.

Vorteilhaft ist es, wenn die Appliziervorrichtung jeweils zwei Griffteile und Flügel sowie entsprechend vier Verrasterungsteile, Rastnasen und Öffnungen im Tragteil vorsieht. Hierdurch werden die Bedienerfreundlichkeit und die Krafteinwirkung parallel zur Kanülenlängsachse beim Lösen der Vorspannvorrichtung erhöht.

Weiterhin vorteilhaft ist es, wenn die Verschlussvorrichtung aus Blech oder aus Kunststoff gebildet ist. Diese Materialien sind preisgünstig und leicht zu bearbeiten. Dabei kann jede denkbare Art der Vorspannung ersonnen werden. Denkbar ist zum Beispiel eine am inneren Rand des Aufnahmeteils angebrachte Druckfeder, die die Verschlussvorrichtung gegen die Kanüle drückt. Denkbar ist auch, das Material der Verschlussvorrichtung vorzuspannen, beispielsweise durch Biegen nach oben, unten oder zur Seite, durch Wölbung oder durch Komprimierung in Längs- oder in Querrichtung.

Auch ist es vorteilhaft, wenn die vorgespannte Verschlussvorrichtung so in dem Aufnahmeteil angeordnet ist, dass sie oberhalb der Bodenöffnung gegen die Kanüle drückt, wobei die Kanüle so an dem Tragteil befestigt ist, dass eine geringe Beweglichkeit gegeben ist. Wenn die Kanüle die Bodenöffnung, in der sie geführt wird, freigibt, kann die vorgespannte Verschlussvorrichtung sie in dem Maße zur Seite drücken, wie dies durch die Elastizität der Befestigung an dem Tragteil möglich ist.

Die Erfindung soll nachfolgend anhand der Figuren 1 bis 7 näher erläutert werden. Es zeigen
- Fig. 1: die Appliziervorrichtung im vorgespannten Zustand
- Fig. 2: die Appliziervorrichtung nach Entfernen des Griffteils beim Lösen der Vorspannvorrichtung
- Fig. 3: die Appliziervorrichtung mit hochgestellten Flügeln
- Fig. 4: die Appliziervorrichtung nach dem Lösen der Vorspannvorrichtung
- Fig. 5: Das Tragteil in Queransicht im vorgespannten Zustand der Appliziervorrichtung
- Fig. 6: die im Aufnahmeteil angeordneten Verrasterungsteile mit Rastnasen
- Fig. 7a: die im Aufnahmeteil angeordnete Verschlussvorrichtung im vorgespannten Zustand
- Fig. 7b: die im Aufnahmeteil angeordnete Verschlussvorrichtung nach dem Verschließen der Bodenöffnung

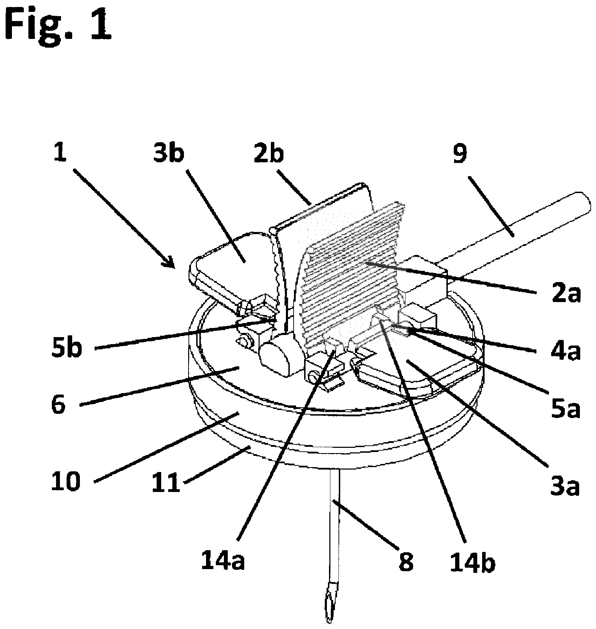

Die Fig. 1 zeigt die Appliziervorrichtung 1 im vorgespannten Zustand. Sie besteht aus einem Tragteil 6, einem Aufnahmeteil 10, einem Bodenteil 11 und einer Kanüle 8. Mit dem Tragteil 6 ist ein Griffteil 2 lösbar verbunden. Das Griffteil 2 kann, wie dargestellt, aus zwei Griffflügeln 2a und 2b bestehen. Diese können miteinander verbunden sein. Alternativ können die Griffflügel 2a und 2b aber auch separate Griffteile sein. Das Griffteil 2 kann jede beliebige, den ergonomischen Anforderungen entsprechende Form haben, also beispielsweise rund, oval oder länglich sein. In Fig. 1 sind die nach außen gerichteten Seiten der Griffflügel 2a und 2b geriffelt, um die Rutschfestigkeit zu erhöhen.

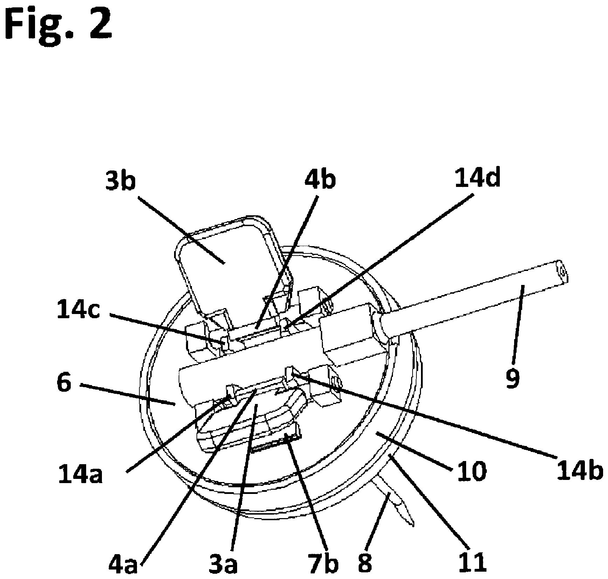

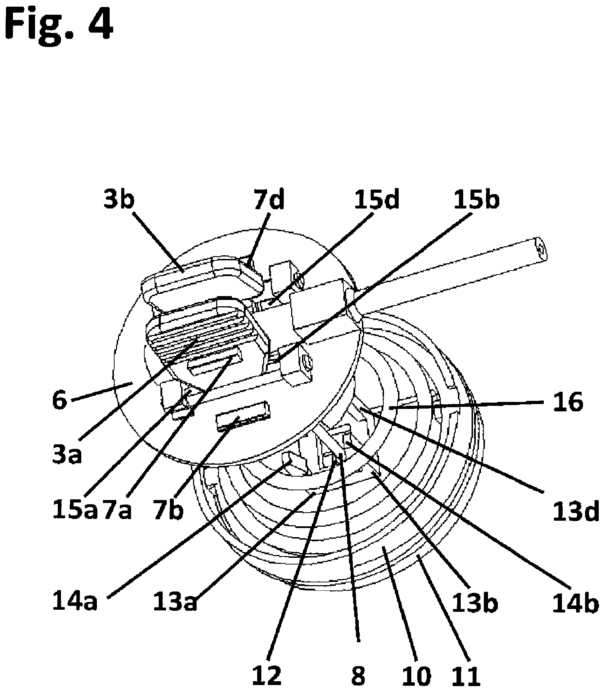

An dem Tragteil 6 ist ferner wenigstens ein Flügel 3 um ein Scharnier 5 drehbar gelagert. Das untere Ende 4 des Flügels 3 ist zwischen zwei Rastnasen 14a, 14b der Vorspannvorrichtung angeordnet. Das Griffteil 2 wirkt so auf das untere Ende 4 des Flügels 3 ein, dass eine Drehbeweglichkeit des Flügels 3 verhindert wird. Fig. 1 zeigt eine Ausführungsform mit zwei Flügeln 3a und 3b und entsprechend zwei Scharnieren 5a und 5b. Die zum Griffteil 2 weisenden unteren Enden 4a und 4b der Flügel 3a und 3b, die zwischen Rastnasen 14a, 14b bzw. - in Fig. 2 zu sehen - 14c, 14d angeordnet sind, werden von den Griffflügeln 2a bzw. 2b des Griffteils 2 an einer Beweglichkeit gehindert, sodass die Flügel 3a und 3b nicht um die Scharniere 5a und 5b drehbar sind. Im vorgespannten Zustand der Appliziervorrichtung 1 werden die Flügel 3a und 3b mit ihren in Fig. 4 sichtbaren Halteteilen 7a und 7c von ebenfalls in Fig. 4 sichtbaren, am Tragteil 6 befestigten Halteteilen 7b und 7d gehalten.

Ein Schlauch 9 wird von dem Tragteil 6 gehalten und ist flüssigkeitsdicht mit der Kanüle 8 verbunden, die an dem Tragteil 6 angeordnet ist.

Fig. 2 zeigt das Tragteil 6 in der Ansicht quer von oben, nachdem das Griffteil 2 entfernt worden ist. Die Flügel 3a, 3b befinden sich in einer Drehbewegung weg von der Oberseite des Tragteils 6 hin zu einer in etwa vertikalen Position parallel zur Längsachse der Kanüle 8. Dabei werden die unteren Enden 4a, 4b der Flügel 3a, 3b nach unten in Richtung auf das Aufnahmeteil 10 verschwenkt. Aus der Oberseite des Tragteils 6 ragen Rastnasen 14a, 14b, 14c und 14d aus in Fig. 4 besser sichtbaren Öffnungen 15a, 15b, 15c und 15d hervor. Die einander zugewandten Seiten der Rastnasen 14a und 14b einerseits sowie 14c und 14d andererseits sind abgeschrägt. In der Drehbewegung drücken das untere Ende 4a des Flügels 3a die Rastnasen 14a und 14b und das untere Ende 4b des Flügels 3b die Rastnasen 14c und 14d auseinander.

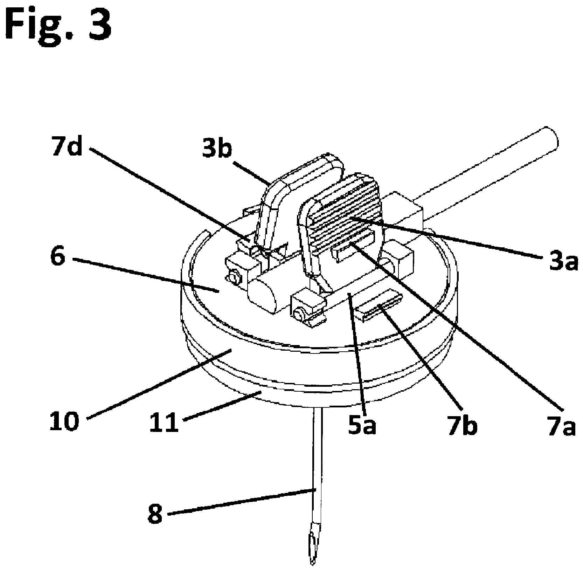

In der Darstellung in Fig. 3 haben die Flügel 3a, 3b ihre in etwa vertikale Position erreicht. Zu erkennen ist jetzt das Halteteil 7a am Flügel 3a, welches im umgeklappten Zustand des Flügels 3a vom Halteteil 7b des Tragteils gehalten wird, z.B. durch Verrasten. Entsprechendes gilt für den Flügel 3b mit seinem nicht dargestellten Halteteil 7c in Bezug auf das am Tragteil 6 angeordnete Halteteil 7d.

Fig. 4 zeigt die Appliziervorrichtung 1, nachdem die Vorspannvorrichtung gelöst worden ist. Die (nicht sichtbaren) unteren Enden 4a, 4b der Flügel 3a, 3b haben die am freien Ende der Verrasterungsteile 13a, 13b, 13c (verdeckt) und 13d angeordneten Rastnasen 14a, 14b, 14c (verdeckt) und 14d über die Öffnungen 15a, 15b, 15c (verdeckt) und 15d des Tragteils 6 verschoben, sodass die Feder 16 das Tragteil 6 jetzt nach oben drücken kann. Die Kanüle 8 wird nunmehr durch die Bodenöffnung 12 des Bodenteils 11 und des Aufnahmeteils 10 in das Innere der Appliziervorrichtung 1 gezogen.

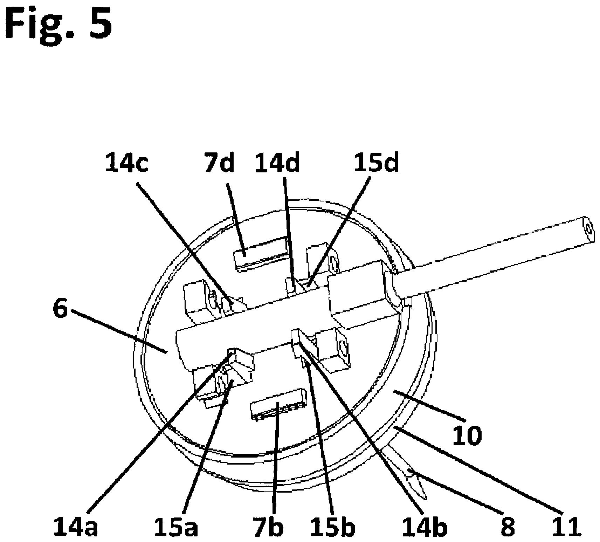

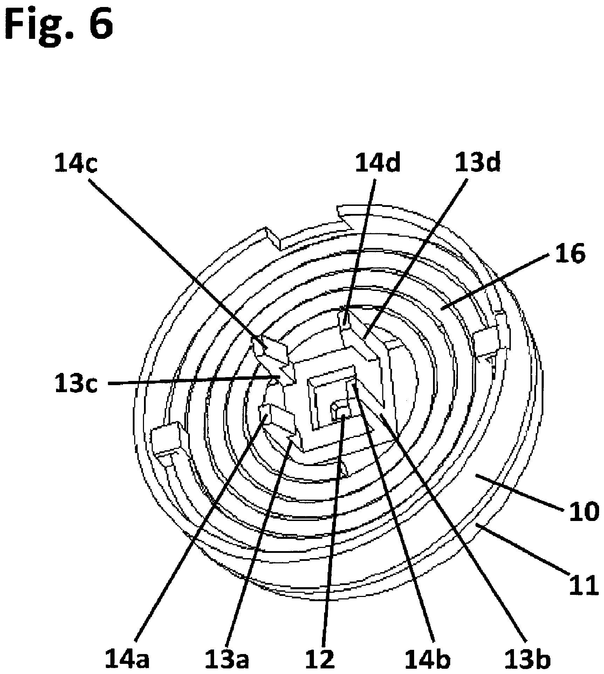

Die Fig. 5 und 6 zeigen die Vorspannvorrichtung. In Fig. 5 sind die Rastnasen 14a, 14b, 14c und 14d zu sehen, die durch die Öffnungen 15a, 15b, 15c (nicht sichtbar) und 15d des Tragteils 6 ragen und auf dem Tragteil 6 aufliegen. In Fig. 6 ist das Aufnahmeteil 10 dargestellt, an dessen Boden die Verrasterungsteile 13a, 13b, 13c und 13d befestigt sind. Am freien Ende der Verrasterungsteile 13a, 13b, 13c und 13d sind die Rastnasen 14a, 14b, 14c und 14d angeordnet. Die einander zugewandten Seiten der Rastnasen 14a und 14b sind abgeschrägt. Gleiches gilt für die Rastnasen 14c und 14d. Die Verrasterungsteile 13a, 13b, 13c und 13d können an ihrem unteren Bereich miteinander verbunden sein, sodass ein Rahmen gebildet wird, der im eingezogenen Zustand der Kanüle 8 ein ungewolltes Ausschlagen der Kanülenspitze verhindert.

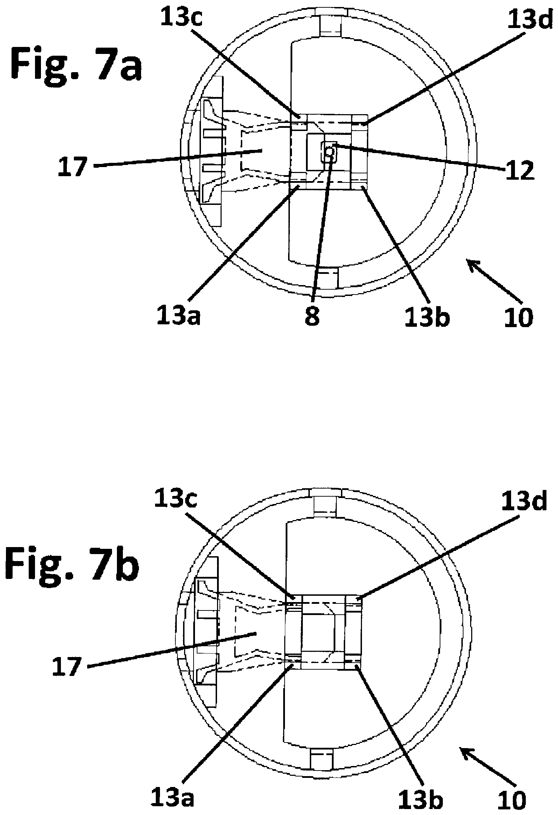

Die Fig. 7a und 7b schließlich zeigen die Verschlussvorrichtung 17 für die Bodenöffnung 12 am Boden des Aufnahmeteils 10. In Fig. 7a ist die Verschlussvorrichtung 17 im vorgespannten Zustand dargestellt. Sie drückt gegen die Kanüle 8 und stellt damit im Zusammenwirken mit der Bodenöffnung 12 eine Führung für die Kanüle 8 dar. In Fig. 7b wird der Zustand gezeigt, der besteht, wenn die Kanüle 8 (nicht dargestellt) durch die Bodenöffnung 12 in das Innere der Appliziervorrichtung 1 gezogen worden ist. Die Verschlussvorrichtung 17 kann sich jetzt entspannen, gleitet über die Bodenöffnung 12 und verschließt sie damit.

### Bezugszeichenliste

- 1: Appliziervorrichtung
- 2a: Griffflügel
- 2b: Griffflügel
- 3a: Flügel
- 3b: Flügel
- 4a: unteres Ende des Flügels 3a
- 4b: unteres Ende des Flügels 3b
- 5a: Scharnier
- 5b: Scharnier
- 6: Tragteil
- 7a: Halteteil
- 7b: Halteteil
- 7c: Halteteil
- 7d: Halteteil
- 8: Kanüle
- 9: Schlauch
- 10: Aufnahmeteil
- 11: Auflageteil
- 12: Bodenöffnung
- 13a: Verrasterungsteil
- 13b: Verrasterungsteil
- 13c: Verrasterungsteil
- 13d: Verrasterungsteil
- 14a: Rastnase
- 14b: Rastnase
- 14c: Rastnase
- 14d: Rastnase
- 15a: Öffnung
- 15b: Öffnung
- 15c: Öffnung
- 15d: Öffnung
- 16: Feder
- 17: Verschlussvorrichtung

## Patentansprüche

1. Appliziervorrichtung (1) für eine Kanüle (8)
- mit wenigstens einem Griffteil (2), das zugleich als Sicherungseinrichtung gegen ein Auslösen der Vorspanneinrichtung dient
- mit einem Tragteil (6)
- mit einem Aufnahmeteil (10)
- mit einem Auflageteil (11)
- mit einer Kanüle (8)
- die Kanüle (8) ragt im vorgespannten Zustand der Appliziervorrichtung (1) durch die Bodenöffnungen (12) des Aufnahmeteils (10) und des Auflageteils (11) hindurch
- die Kanüle (8) ist an dem Tragteil (6) befestigt
- mit dem Tragteil (6) ist das wenigstens eine Griffteil (2) lösbar verbunden
- an dem Tragteil (6) ist wenigstens ein Flügel (3) drehbeweglich angeordnet
- in der Nichtgebrauchsstellung ist der wenigstens eine Flügel (3) durch sein Halteteil (7a) mit dem Halteteil (7b) des Tragteils lösbar verbunden, wobei das Griffteil (2) eine Drehbeweglichkeit des Flügels (3) verhindert
- das Tragteil (6) ist in einem Aufnahmeteil (10) angeordnet
- das Aufnahmeteil (10) weist wenigstens zwei Verrasterungsteile (13a,13b) auf, die senkrecht vom Boden des Aufnahmeteils (10) aufragen
- die oberen Enden der Verrasterungsteile (13a,13b) weisen einander zugewandte Rastnasen (14a,14b) auf
- die einander zugewandten Seiten der Rastnasen (14a,14b) sind abgeschrägt
- das Tragteil (6) ist durch eine Feder (16) gegen das Aufnahmeteil (10) vorgespannt
- in dem vorgespannten Zustand ragen die wenigstens zwei Rastnasen (14a,14b) durch wenigstens zwei Öffnungen (15a,15b) des Tragteils (6) hindurch und liegen auf der Oberseite des Tragteils (6) auf
- in dem vorgespannten Zustand ist das zur Mitte des Tragteils (6) weisende untere Ende (4) des wenigstens einen Flügels (3) zwischen den abgeschrägten Seiten der wenigstens zwei Rastnasen (14a,14b) angeordnet
- nach Entfernen des wenigstens einen Griffteils (2) wird der wenigstens eine Flügel (3) um das Scharnier (5) drehbeweglich, wobei durch die Drehbewegung das Halteteil (7a) des wenigstens einen Flügels (3) von dem Halteteil (7b) des Tragteils (6) gelöst wird
- In einer vertikalen Position des Flügels (3) drückt sein zur Mitte des Tragteils (6) weisendes unteres Ende (4) die Rastnasen (14a,14b) auseinander, sodass diese nicht mehr auf der Oberseite des Tragteils (6) aufliegen, wodurch die Vorspannvorrichtung gelöst wird
- durch das Lösen der Vorspannvorrichtung wird das Tragteil (6) durch die Feder (16) vom Aufnahmeteil (10) weg nach oben gedrückt, wodurch die Kanüle (8) vollständig in den Raum zwischen Tragteil (6) und Aufnahmeteil (10) gezogen wird
- in dem Aufnahmeteil (10) ist eine vorgespannte Verschlusseinrichtung (17) angeordnet, die durch Federdruck die Bodenöffnung (12) des Aufnahmeteils (10) verschließt, sobald die Kanüle (8) durch die Bodenöffnungen (12) des Auflageteils (11) und des Aufnahmeteils (10) hindurch gezogen worden ist.

2. Appliziervorrichtung (1) nach Anspruch 1,
- mit zwei Griffteilen (2a,2b)
- mit zwei Flügeln (3a,3b)
- mit vier Verrasterungsteilen (13a,13b,13c,13d)
- mit vier Rastnasen (14a,14b,14c,14d)
- mit vier Öffnungen (15a,15b,15c,15d) im Tragteil (6).

3. Appliziervorrichtung (1) nach einem der vorangegangenen Ansprüche, bei der die Verschlussvorrichtung (17) als vorgespanntes Blech oder als vorgespanntes Kunststoffteil ausgebildet ist.

4. Appliziervorrichtung (1) nach einem der vorangehenden Ansprüche, bei der die vorgespannte Verschlussvorrichtung (17) gegen die Kanüle (8) drückt und das untere Ende der Kanüle (8), sobald diese die Bodenöffnung (12) freigegeben hat, so zur Seite drückt, dass ein neuerliches Hindurchtreten der Kanülenspitze durch die Bodenöffnung (12) verhindert wird.

## Claims

1. - Applicator for a cannula
- with at least one handle part, which at the same time serves as a safety device against the triggering of the preloading device
- with one needle seat
- with one spring seat
- with one pad
- with one cannula
- In a preloaded state of the applicator the cannula protrudes from the bottom openings of the spring seat and the pad.
- The cannula is fixed to the needle seat.
- The at least one handle part is connected with the needle seat in a detachable way.
- At least one wing on the needle seat is arranged for rotatable movement.
- In the not-in-use position the at least one wing is detachably connected by its holding part with the holding part of the needle seat, the handle part preventing the wing from rotating.
- The needle seat is arranged in a spring seat
- The spring seat has got at least two snap-in parts rising vertically from the bottom of the spring seat.
- The top ends of the snap-in parts show snap-in noses facing each other.
- The sides of the snap-in noses facing each other are bevelled.
- The needle seat is spring loaded against the spring seat.
- In the preloaded state, the at least 2 snap-in noses protrude through at least 2 openings of the needle seat and rest on the upper side of the needle seat.
- In the preloaded state, the lower end of the at least one wing pointing towards the centre of the needle seat is located between the bevelled sides of the at least two snap-in noses.
- After removal of the at least one handle part, the at least one wing becomes rotationally movable about the hinge, whereby the rotational movement releases the holding part of the at least one wing from the holding part of the needle seat.
- In a vertical position of the wing, its lower end facing the centre of the needle seat presses the snap-in noses apart so that they no longer rest on the upper side of the needle seat, thus releasing the preloaded device, so that they no longer rest on the upper side of the needle seat, thereby releasing the preloaded device.
- By releasing the preloaded device, the needle seat is pushed upwards by the spring away from the spring seat, whereby the cannula is pulled completely into the space between needle seat and spring seat.
- A preloaded closing device is arranged in the spring seat, which closes the bottom opening of the spring seat by spring pressure as soon as the cannula has been pulled through the bottom openings of the needle seat and the spring seat.

2. An applicator according to claim 1,
- with two handle parts
- two-winged
- with four locking parts
- with four snap-in noses
- with four openings in the needle seat

3. An applicator according to any of the foregoing claims, in which the closure device is formed as a preloaded sheet or as a preloaded plastic part.

4. An applicator according to any of the foregoing claims, in which the preloaded closure device presses against the cannula and, once the cannula has released the bottom opening presses the lower end of the cannula sideways so as to prevent the tip of the cannula from passing through the bottom opening again.

## Revendications

1. Applicateur de canule
- avec au moins une partie poignée, qui sert en même temps de dispositif de sécurité contre le déclenchement du dispositif de précontrainte
- avec une pièce de support
- avec une pièce réceptrice
- avec une pièce d'appui
- avec une canule
- A l'état précontraint de l'applicateur, la canule dépasse les ouvertures inférieures de la pièce réceptrice et de la pièce d'appui.
- La canule est fixée à la pièce de support.
- La partie poignée - dont il existe au moins une - est reliée de manière amovible à la pièce de support.
- La pièce de support dispose d'au moins une ailette rotative.
- En position de non-utilisation, sa pièce de maintien relie l'ailette - dont il existe au moins une - de manière amovible à la pièce de maintien de la pièce de support, la partie poignée empêchant la rotation de l'ailette.
- La pièce de support est disposée dans une pièce réceptrice.
- La pièce réceptrice comporte au moins deux pièces de verrouillage qui se dressent perpendiculairement sur le fond de la pièce réceptrice.
- Les extrémités supérieures des pièces de verrouillage ont des ergots d'encliquetage disposés l'un en face de l'autre.
- Les côtés des ergots d'encliquetage qui se font face sont biseautés,
- La pièce de support est précontrainte contre la pièce réceptrice à l'aide d'un ressort.
- À l'état précontraint, les ergots d'encliquetage - dont il existe au moins deux - dépassent des ouvertures - dont il y a au moins deux - de la pièce de support et reposent sur la partie supérieure de la pièce de support.
- À l'état précontraint, l'extrémité inférieure de l'ailette - dont il existe au moins une -orientée vers le centre de la pièce de support est située entre les côtés biseautés des au moins deux ergots d'encliquetage.
- Le retrait de la partie poignée - dont il y a au moins une - permet à l'ailette - dont il y a au moins une - de tourner autour de la charnière, ce mouvement de rotation libérant la pièce de maintien de l'ailette de la pièce de maintien de la pièce de support.
- Lorsque l'ailette est en position verticale, son extrémité inférieure orientée vers le centre de la pièce de support écarte les ergots de verrouillage de manière à ce qu'ils ne reposent plus sur la partie supérieure de la pièce de support, ce qui déclenche le dispositif de précontrainte.
- Le dispositif de précontrainte déclenché, le ressort pousse la pièce de support vers le haut et l'éloigne de la pièce réceptrice, ce qui a pour effet que la canule entre entièrement dans l'espace entre la pièce de support et la pièce réceptrice.
- La pièce réceptrice comporte un dispositif de fermeture précontraint, qui ferme l'ouverture inférieure de la pièce réceptrice par la pression d'un ressort dès que la canule a traversé les ouvertures inférieures de la pièce d'appui et de la pièce réceptrice.

2. Applicateur selon la revendication 1,
- avec deux parties poignées
- avec deux ailettes
- avec quatre pièces de verrouillage
- avec quatre ergots d'encliquetage
- avec quatre ouvertures dans la pièce de support.

3. Applicateur selon l'une des revendications précédentes, dans lequel le dispositif de fermeture est conçu comme une tôle précontrainte ou comme une pièce en plastique précontrainte.

4. Applicateur selon l'une des revendications précédentes, dans lequel le dispositif de fermeture précontraint exerce une pression sur la canule et pousse l'extrémité inférieure de la canule vers le côté dès que la canule a libéré l'ouverture inférieure, empêchant ainsi la pointe de la canule de passer à nouveau par l'ouverture inférieure.
